(19) Europäisches Patentamt
European Patent Office
Office européen des brevets

(11) Veröffentlichungsnummer: **0 305 721 B1**

(12) # EUROPÄISCHE PATENTSCHRIFT

(45) Veröffentlichungstag der Patentschrift: **16.10.91**

(51) Int. Cl.[5]: **C07C 279/28**, C07C 277/02

(21) Anmeldenummer: **88111828.5**

(22) Anmeldetag: **22.07.88**

(54) **Verfahren zur Herstellung von 1,6-Di(N3-cyano-N1-guanidino)hexan.**

(30) Priorität: **04.09.87 DE 3729530**

(43) Veröffentlichungstag der Anmeldung:
**08.03.89 Patentblatt 89/10**

(45) Bekanntmachung des Hinweises auf die Patenterteilung:
**16.10.91 Patentblatt 91/42**

(84) Benannte Vertragsstaaten:
**AT BE CH DE ES FR GB IT LI NL SE**

(56) Entgegenhaltungen:
**EP-A- 0 125 092**
**DE-B- 1 443 386**
**GB-A- 705 838**
**DE-A- 2 932 951**
**DE-B- 1 568 581**
**US-A- 4 537 746**

(73) Patentinhaber: **Degussa Aktiengesellschaft**
**Weissfrauenstrasse 9**
**W-6000 Frankfurt am Main 1(DE)**

(72) Erfinder: **Huthmacher, Klaus, Dr.**
**Lärchenweg 18**
**W-6460 Gelnhausen(DE)**
Erfinder: **Bethge, Horst**
**Schwalbenstrasse 2a**
**W-6450 Hanau(DE)**
Erfinder: **Kleeman, Axel, Prof.**
**Bornweg 36**
**W-6052 Mühlheim 2(DE)**
Erfinder: **Braun, Rolf**
**Herzbergstrasse 56**
**W-6466 Gründau(DE)**

**EP 0 305 721 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung richtet sich auf ein verbessertes Verfahren zur Herstellung von 1,6-Di(N$^3$-cyano-N$^1$-guanidino)hexan aus einem Alkalidicyanamid und einem Hexamethylendiammoniumsalz. Das Verfahren ist auch im Betriebsmaßstab einfach durchführbar und zeichnet sich insbesonders durch verkürzte Reaktionszeiten und erhöhte Raum-Zeit-Ausbeuten aus, ferner wird ein sehr reines Produkt in hoher Ausbeute erhalten.

1,6-Di(N$^3$-cyano-N$^1$-guanidion)hexan, oft als Hexamethylen-bis-cyanoguanidin bzw. Hexamethylen-bis-dicyandiamid bezeichnet, ist ein .wertvolles Zwischenprodukt zur Herstellung von Bisbiguaniden und Polybiguaniden, die als Desinfektionsmittel oder Schädlingsbekämpfungsmittel eingesetzt werden (siehe beispielsweise GB-PS 705 838, EP 125091; EP 125092; EP 125093; EP 126567; EP 127062).

Eines der wichtigsten Folgeprodukte von 1,6-Di(N$^3$-cyano-N$^1$-guanidino)hexan ist 1,6-Di(N$^5$-p-chlorphenyl-N$^1$-diguanido)-hexan, allgemein als Chlorhexidin bezeichnet. Chlorhexidin ist ein stark wirksames antibakterielles und antiseptisches Mittel gegen grampositive und gramnegative Bakterien.

Zur Herstellung von 1,6-Di(N$^3$-cyano-N$^1$-guanidino)hexan sind verschiedene Verfahren beschrieben worden:

F.L. Rose und G. Swain (J. Chem. Soc. (1956), 4422 -4425) setzen Natriumdicyanamid und Hexamethylendiamin-dihydrochlorid in stöchiometrischem Verhältnis in n-Butanol durch 8-stündiges Erhitzen unter Rückfluß gemäß nachstehender Gleichung miteinander um:

$$2\ Na\ N(CN)_2 \ + \ H_2N-(CH_2)_6-NH_2 \cdot 2\ HCl \longrightarrow$$

$$NC-NH-\underset{\underset{NH}{\|}}{C}-NH-(CH_2)_6-NH-\underset{\underset{NH}{\|}}{C}-NH-CN \quad + \quad 2\ NaCl$$

(I)

Das beim Abkühlen auskristallisierende Rohprodukt des gewünschten 1,6-Di(N$^3$-cyano-N$^1$-guanidino)hexan (I) wird zusammen mit ungelöst vorliegendem gebildeten Kochsalz abfiltriert und anschließend mit Wasser gewaschen und dann getrocknet. Die Ausbeute an (I) soll 70 bis 80 % betragen. Über die Reinheit von (I) wird nichts ausgesagt, jedoch ist der angegebene Schmelzpunkt des bereits aus Wasser umkristallisierten Produktes (202 - 203 °C) immer noch deutlich unter demjenigen eines reinen Produktes (209 - 210 °C). Von der Anmelderin nach Angaben dieses Dokumentes durchgeführte Versuche haben Gehalte an (I) von ca. 90 % ergeben. Nachteilig an diesem bekannten Verfahren ist aber nicht nur die geringe Produktreinheit, welche sich in einer verringerten Ausbeute bei der Umsetzung zu Chlorhexidin bemerkbar macht, sondern auch die mäßige Ausbeute. Darüber hinaus führen der apparative Aufwand und die lange Reaktionszeit zu einer niedrigen Raum-Zeit-Ausbeute.

In der US-PS 4,537,746 wird ein Beispiel zur Herstellung von 1,6-Di(N$^3$-cyano-N$^1$-guanidino)hexan (I) nach dem Verfahren von Rose et al beschrieben, wobei konkret auf das oben gewürdigte Dokument verwiesen wird.

Das Rohprodukt (I) mit einem Schmelzpunkt von 200 -203 °C muß dabei aus einem sehr großen Volumen Methanol-Wasser-Gemisch umkristallisiert werden, um eine für die Folgeumsetzungen zu Desinfektionsmitteln erforderliche Produktqualität zu erhalten. Die Anmelderin hat das Beispiel nachgearbeitet, anstelle der angegebenen 94 %igen Ausbeute aber nur eine solche von 45 % (63 % Rohprodukt) erhalten; ferner betrug die Produktreinheit nach Umkristallisation nur 93,3%. Das Beispiel der US-PS 4,537,746 macht keine Angabe zur Qualität des eingesetzen Natriumdicyanamids -1 Mol entspricht 89 g, eingesetzt wurden aber 103 g.

J. Burns (J. Labelled Comp. Radiopharm. 19, (1982), 1239 - 1250) lehrt ein Verfahren zur Herstellung von (I), wobei die bereits bei den vorgenannten Verfahren eingesetzten Stoffe miteinander umgesetzt werden, Natriumdicyanamid in geringem Überschuß eingesetzt und über Molekularsieb getrocknetes Isopropanol als Lösungsmittel verwendet wird. Dieses Verfahren erfordert eine 16stündige Reaktionszeit und macht (1) in 57 %iger Ausbeute zugänglich.

Gemäß der DE-OS 29 32 951 läßt sich 1,6-Di(N$^3$-cyano-N$^1$-guanidino)hexan auch durch die Umsetzung von Hexamethylendiamin mit einem N-Cyano-O (oder S)-alkyliso (oder isothio)-harnstoff herstellen. Die Herstellung der Harnstoff-Ausgangskomponente verläuft über die Umsetzung von O- bzw. S-Estern der N-Cyanoiminokohlensäure mit Ammoniak oder einem Ammoniumcarbonat; die Umsetzung mit Hexamethylendiamin erfolgt unter Druck und erfordert eine sehr lange Reaktionszeit. Das erhaltene Produkt muß

nachträglich mit Wasser und Alkohol gereinigt werden. Nachteile dieses und ähnlicher Verfahren (vgl. HU-PS 17484 und 15453) sind das Arbeiten unter Druck, die sehr langen Reaktionszeiten, die Bildung von Mercaptanen beim Einsatz von Isothioharnstoffen, der Einsatz teurer Ausgangsstoffe und der Reinigungsaufwand für das gewünschte Endprodukt (I).

Die Aufgabe der Erfindung liegt in der Bereitstellung eines verbesserten Verfahrens zur Herstellung von 1,6-Di($N^3$-cyano-$N^1$-guanidino)hexan aus einem Alkali-dicyanamid und einem Hexamethylendiammoniumsalz, wie Hexamethylendiamin-dihydrochlorid. Das Verfahren sollte sich einfach, zuverlässig und mit hoher Wirtschaftlichkeit im Betriebsmaßstab durchführen lassen. Ferner sollte das Verfahren das Produkt in hoher Ausbeute und in einer solchen Reinheit verfügbar machen, welche eine weitere Umsetzung, zu beispielsweise Chlorhexidin, ohne Umkristallisation oder andere aufwendige Reinigung ermöglicht.

Überraschenderweise und im Gegensatz zu vorbekannten Verfahren, welche die Umsetzung in einem wasserfreien Alkohol durchführen, wurde nun gefunden, daß sich die Umsetzung durch die Anwesenheit einer begrenzten Menge Wasser und einer katalytischen Menge einer Base beschleunigen läßt, und eine hohe Ausbeute erhalten wird, wenn der pH-Wert zu Beginn der Umsetzung 8 bis 10 beträgt. Unerwartet war die weitere Feststellung, daß durch Zugabe von Wasser zum noch erwärmten Reaktionsgemisch ein leicht filtrierbares Reaktionsprodukt in höherer Reinheit gewonnen werden kann, als dies nach den vorbekannten Verfahren mit aufwendiger Umkristallisation möglich war.

Die Aufgabe der Erfindung wird also gelöst durch ein Verfahren zur Herstellung von 1,6-Di($N^3$-cyano-$N^1$-guanidino)hexan durch Umsetzung eines Alkalidicyanamids mit einem Hexamethylendiammoniumsalz in alkoholischer Lösung bzw Suspension unter Erwärmen auf mindestens 80 °C, Abkühlen des Reaktionsgemisches nach beendeter Umsetzung, Abtrennen des auskristallisierten und durch Behandlung mit Wasser im wesentlichen salzfrei gemachten Reaktionsproduktes und Trocknen desselben, das dadurch gekennzeichnet ist, daß man die Umsetzung in Gegenwart von 0,1 bis 20 Gew.-% Wasser, bezogen auf das im Reaktionsgemisch anwesende Lösungsmittel einschließlich Wasser, und einer katalytischen Menge einer Base aus der Reihe aliphatisches oder cycloaliphatisches tertiäres Amin, N-heterocyclische Base oder Hexamethylendiamin durchführt, wobei eine solche Menge Base eingesetzt wird, daß das die Reaktionspartner in stöchiometrischem Verhältnis enthaltende Reaktionsgemisch zu Beginn der Umsetzung einen pH-Wert von 8 bis 10 - gemessen mit einer Glaselektrode bei etwa 25 °C - aufweist, nach beendeter Umsetzung das Reaktionsgemisch oberhalb 80 °C mit Wasser versetzt und das auskristallisierte Reaktionsprodukt von der salzhaltigen flüssigen Phase abtrennt.

Die Ansprüche 2 bis 13 richten sich auf bevorzugte Ausführungsformen des erfindungsgemäßen Verfahrens.

Die im Reaktionsgmisch zu Beginn der Umsetzung enthaltenen Salze Alkalidicyanamid und Hexamethylendiammoniumsalz sowie das entstehende Natriumsalz liegen in der alkoholischen Lösung überwiegend in suspendierter Form vor. Es wird angenommen, daß durch die erfindungsgemäße Gegenwart von Wasser die Salze in gewissem Umfang gelöst werden und/oder in eine sehr feine Suspension überführt werden, wodurch die Reaktion zum gewünschten Produkt erleichtert wird. Daß erfindungsgemäß andererseits die Ausbeute nicht sinkt, sondern sogar sehr hoch ist - im allgemeinen um 85 - 95 % der Theorie - war überraschend, weil Wasser das eingesetzte Alkalidicyanamid hydrolysieren kann. Im Wasser als Lösungsmittel kann daher 1,6-Di($N^3$-cyano-$N^1$-guanidino)hexan durch Umsetzung von z.B. Natriumdicyanamid mit Hexamethylendiamin-dihydrochlorid nicht erhalten werden. Aus dem gleichen Grunde ist auch die im erfindungsgemäßen Verfahren eingesetzte Menge Wasser begrenzt, und zwar auf 0,1 bis 20 Gew.-%, bezogen auf das im Reaktionsgemisch anwesende Lösungsmittel einschließlich Wasser. Die Hydrolysegefahr nimmt mit zunehmender Menge Wasser im Reaktionsgemisch und zunehmender Temperatur zu, weshalb bei Temperaturen um/über 100 °C ein Wassergehalt von 0,5 bis 5 Gew.-%, gleiche Bezugsbasis wie oben, vorteilhaft ist. Ein zu geringer Wassergehalt führt aber zu einer Verlangsamung der Umsetzung und, offensichtlich bedingt durch die längere Reaktionszeit, zu einer Ausbeuteminderung.

In einer bevorzugten Ausführungsform erfolgt die Herstellung des Reaktionsgemisches in der Weise, daß eine wäßrige, gegebenenfalls wäßrig-alkoholische Lösung von Hexamethylendiamin mit wäßriger Salzsäure versetzt wird, wobei sich Hexamethylendiamin-dihydrochlorid bildet; die wäßrige Lösung mit einem pH-Wert zwischen 5 und 6 wird sodann, falls nicht bereits zugegen, mit dem Alkohol, dem Alkalidicyanamid und dem Katalysator zusammengebracht. Die wäßrige Hexamethylendiamin-dihydrochlorid-Lösung kann direkt der alkoholischen Suspension eines Alkalidicyanamids zugegeben werden, wobei die Temperatur der Suspension im Bereich von Raumtemperatur bis etwa 100 °C liegen kann. Es ist auch möglich, umgekehrt zu verfahren, d.h. eine wäßrig-alkoholische sehr feine, milchig aussehende Suspension von in situ gebildetem Hexamethylendiamin-dihydrochlorid vorzulegen und Alkalidicyanamid einzutragen und durch Zugabe der katalytisch wirksamen Base den pH-Wert einzustellen.

Während bei der zuvor dargelegten Ausführungsform Wasser durch die verwendete Salzsäure und

gegebenenfalls Hexamethylendiamin, das im Handel als 90 %ige wäßrige Lösung preisgünstig zugänglich ist, in das Reaktionsgemisch eingetragen wird, kann dies ganz oder teilweise auch durch das verwendete alkoholische Lösungsmittel und/oder Feuchte der eingesetzten Reaktionsparter bzw. Base und/oder durch direkte Zugabe erfolgen. Die Möglichkeit, ein feuchtes alkoholisches Lösungsmittel einsetzen zu können, ist gegenüber den vorbekannten Verfahren ein wesentlicher Vorteil, weil zurückgewonnenes Lösungsmittel nicht getrocknet werden muß.

Unter der Formulierung "zu Beginn der Umsetzung" wird der Zeitpunkt verstanden, wenn das Reaktionsgemisch alle für die Umsetzung erforderlichen Bestandteile enthält, unabhängig davon, ob die Temperatur des Gemisches dabei Raumtemperatur oder erhöhte Temperatur aufweist.

Nach Zubereitung des Reaktionsgemisches wird dieses auf die gewünschte Reaktionstemperatur im Bereich von etwa 80 °C bis 170 °C, bevorzugt 110 °C bis 150 °C, erwärmt und solange bei etwa dieser Temperatur gehalten, meist unter Rühren, bis die Umsetzung beendet ist. Das Ende läßt sich anhand eines Tests unter Verwendung gaschromatographischer Analysenmethoden leicht feststellen.

Insbesondere dann, wenn durch die Zubereitung des Reaktionsgemisches eine höhere Konzentration Wasser zugegen ist, d.h. eine solche im Bereich von etwa 5 bis 20 Gew.-%, bezogen auf das anwesende Lösungsmittel einschließlich Wasser, ist es vorteilhaft, während des Erwärmens auf die Reaktionstemperatur, die Konzentration an Wasser durch Abdestillieren desselben auf 5 bis 0,5 Gew.-% zu erniedrigen. Das Abdestillieren kann dabei als Azeotrop mit dem Alkohol erfolgen und bei Normaldruck oder/und vermindertem Druck erfolgen. Sofern beispielsweise eine wäßrige Hexamethylendiamin-dihydrochlorid-Lösung bei einer Temperatur um oder oberhalb 80 °C in eine Alkalidicyanamid enthaltende Suspension eingetragen wird, kann bereits während der Zubereitung des Reaktionsgemisches ein Teil des Wassers abdestilliert werden.

Das die Reaktionspartner in stöchiometrischem Verhältnis enthaltende Reaktionsgemisch weist zu Beginn der Umsetzung einen pH-Wert im Bereich 8 bis 10 auf. Gemessen wird der pH-Wert in üblicher Weise, beispielsweise mit einer Glaselektrode. Der Bereich pH 8 bis 10 bezieht sich auf eine Meßtemperatur um etwa 25 °C, was aber nicht bedeutet, daß zu Beginn der Umsetzung das Reaktionsgemisch eine solche Temperatur aufweisen müßte. Ein pH-Wert außerhalb pH 8 bis 10 zu Beginn der Umsetzung führt zu einer Ausbeuteminderung. Ein pH-Wert im Bereich um 9 bis 9,5 zu Beginn der Umsetzung wird bevorzugt, weil in diesem Bereich im allgemeinen die Produktausbeute am höchsten ist.

Der gewünschte pH-Wert wird durch die Anwesenheit einer katalytischen Menge einer Base im Reaktionsgemisch eingestellt. Die Base kann dabei dem die übrigen Bestandteile enthaltenden Reaktionsgemisch in einer solchen Menge direkt zugegeben werden, daß bei einer Meßtemperatur um 25 °C ein pH-Wert von 8 bis 10 resultiert. Alternativ kann die Base auch ganz oder teilweise mit dem verwendeten alkoholischen Lösungsmittel eingebracht werden. Bei einer bevorzugten Ausführungsform, beispielsweise bei Verwendung von n-Butanol als Lösungsmittel und Triethylamin als Base, wird während und/oder nach der Umsetzung ein Gemisch des feuchten alkoholischen Lösungsmittels gemeinsam mit dem Amin abdestilliert und als solches dem folgenden Ansatz zugeführt. Sofern das Destillat zwei Phasen bildet - wäßrig und organisch - , werden die Phasen voneinander getrennt und die organische Phase dem nächsten Ansatz zugeführt.

Als Base werden aliphatische oder cycloaliphatische Amine, N-heterocyclische Basen oder Hexamethylendiamin zur pH-Einstellung verwendet. Bevorzugte Verbindungen aus den genannten Verbindungsklassen enthalten 5 bis 7 C-Atome und ein oder zwei N-Atome, wie beispielsweise Triethylamin, N-Methyl-Morpholin oder Pyridin.

Die zuvor erörterten Merkmale des erfindungsgemäßen Verfahrens führen zu einer beschleunigten Umsetzung des Alkalidicyanamids mit dem Hexamethylendiammoniumsalz. Gegenüber den bisher bekannten, auf der gleichen Umsetzung beruhenden Verfahren ist es nun möglich, die Reaktionszeit zu verkürzen; in bevorzugten Ausführungsformen bedeutet dies mehr als eine Halbierung der Reaktionszeit. Überraschenderweise wird im allgemeinen zusätzlich die Ausbeute gesteigert. Durch die Verkürzung der Reaktionszeit und damit Erhöhung der Raum-Zeit-Ausbeute sowie die Ausbeutesteigerung wird das Verfahren überaus wirtschaftlich. Dieser Vorteil wird in der Ausführungsform der in situ-Bildung von Hexamethylendiamin-dihydrochlorid aus Hexamethylendiamin und wäßriger Salzsäure weiter gesteigert, weil es nicht mehr erforderlich ist, das teure Salz direkt einzusetzen. Zusätzlich können auch das Alkalidicyanamid und Lösungsmittel in feuchtem Zustand eingesetzt werden, wodurch Kosten für eine Trocknung gespart werden.

Ein weiteres, die Wirtschaftlichkeit des Verfahrens steigerndes Merkmal richtet sich auf die Aufarbeitung nach beendeter Umsetzung. Die Filtration des aus dem alkoholischen Lösungsmittel auskristallisierten 1,6-Di($N^3$-cyano-$N^1$-guanidino)hexans und Auswaschen des mitangefallenen Alkalisalzes mit Wasser und die Umkristallisation waren bei den bekannten Verfahren sehr zeitaufwendig und begrenzten ganz wesentlich die Anlagenkapazität; gleichzeitig war das Reaktionsprodukt salzhaltig und/oder anderweitig verunreinigt.

Erfindungsgemäß werden diese Probleme behoben, indem das Reaktionsgemisch nach beendeter Umsetzung, vorzugsweise nach Abdestillieren eines Teils des Lösungsmittels, oberhalb 80 °C mit Wasser versetzt, dabei oder anschließend abgekühlt und dann das auskristallisierte Produkt von der salzhaltigen flüssigen Phase abgetrennt wird. Hierbei löst sich das gebildete Salz vollständig in der flüssigen Phase, und das kristalline 1,6-Di($N^3$-cyano-$N^1$-guanidino)hexan (I) fällt in reiner, grobkristalliner und damit leicht filtrierbarer Form an. Nach Zugabe von ausreichend Wasser kann das alkoholische Lösungsmittel, soweit es mit Wasser Azeotrope bildet oder unter 100 °C bei Normaldruck siedet, abdestilliert werden. Bevorzugt verwendet man solche Alkohole, welche mit Wasser Azeotrope bilden und über 100 °C sieden, und trennt diese durch Azeotropdestillation vor der Fest-Flüssig-Phasentrennung ab. (I) enthält praktisch keine salzhaltigen Einschlüsse, und die Restfeuchte ist sehr gering, wenn die Phasentrennung in üblichen Fest-Flüssig-Trennvorrichtungen, wie Filter oder Zentrifugen, vorgenommen wird. (I) fällt somit sehr rein an, im allgemeinen über 95 %. Eine sofortige weitere Umsetzung kann ohne aufwendige Reinigung, wie Umkristallisation, vorgenommen werden.

In einer bevorzugten Ausführungsform wird nach beendeter Umsetzung das Reaktionsgemisch oberhalb 80 °C, im allgemeinen beginnend bei der zuletzt herrschenden oder nur mäßig erniedrigten Reaktionstemperatur, mit Wasser versetzt. Nach gegebenenfalls erfolgter Entfernung von Lösungsmittel, durch zum Beispiel Azeotropdestillation, wird auf 25 - 60 °C, vorzugsweise 30 - 50 °C, abgekühlt. Die Phasentrennung wird anschließend bei dieser Temperatur vorgenommen. Vor der Zugabe des Wassers, gegebenenfalls auch während der Umsetzung selbst, wird vorzugsweise ein Teil des Lösungsmittels, oft gemeinsam mit der verwendeten Base, abdestilliert. Die Menge Lösungsmittel, welche zu Beginn der Wasserzugabe noch anwesend ist und welche Menge Wasser zugesetzt wird, wird der Fachmann durch Vorversuche ermitteln. Sofern das verwendete alkoholische Lösungsmittel vor der Fest-Flüssig-Phasentrennung nicht aus der flüssigen Phase entfernt wird, muß es im Anschluß an diese aus der in diesem Falle wäßrig-alkoholischen Phase zurückgewonnen werden, z.B. durch Aussalzen oder Extraktion. Erfindungswesentlich ist die Zugabe des Wassers in der Wärme, oberhalb 80 °C, bevorzugt oberhalb 110 °C, weil dadurch die Ausbildung grober Kristalle des Reaktionsproduktes ermöglicht wird. Die Raum-Zeit-Ausbeute für die Phasentrennung konnte durch die so möglich gewordene gute und rasche Filtrierbarkeit vermehrfacht werden.

Unter den Alkalidicyanamiden wird Natriumdicyanamid bevorzugt. Natriumdicyanamid ist im Handel erhältlich. Wie gesagt, kann auch feuchtes Natriumdicyanamid verwendet werden.

Bevorzugt verwendetes Hexamethylendiammoniumsalz ist das Hexamethylendiamin-dihydrochlorid (1,6-Diaminohexan-dihydrochlorid). Grundsätzlich können auch andere Salze von 1,6-Diaminohexan mit bevorzugt starken Säuren - ein Äquivalent Säure pro Aminogruppe - zum Einsatz gelangen.

Gemäß bevorzugten Ausführungsformen wird Natriumdicyanamid mit Hexamethylendiamin-dihydrochlorid, bevorzugt aus Hexamethylendiamin und Salzsäure in situ gebildet, eingesetzt.

Die alkoholische Lösung für die Umsetzung enthält als Lösungsmittel einen oder mehrere mono- oder divalente Alkohole. Bevorzugt sind Alkohole mit 1 bis 6 C-Atomen, besonders primäre Alkohole. Ganz besonders vorteilhaft ist die Verwendung eines monovalenten primären Alkohols mit 3 bis 5 C-Atomen, wie n-Butanol. Besonders bevorzugte Alkohole sieden im Bereich von etwa 100 °C bis 160 °C. Bei unter der gewünschten Reaktionstemperatur siedenden Alkoholen kann die Umsetzung bei erhöhtem Druck durchgeführt werden, was aber aufwendiger ist. Der verwendete Alkohol kann auch andere funktionelle Gruppen, wie z.B. Methoxy, enthalten, soweit diese unter den Reaktionsbedingungen stabil sind. Die alkoholische Lösung enthält als Lösungsmittel im wesentlichen Alkohole und im allgemeinen 0,1 bis 20 Gew.-% Wasser, bezogen auf Lösungsmittel und Wasser; es können in begrenztem Umfang aber auch andere organische Lösungsmittel, welche die Umsetzung nicht negativ beeinflussen, zugegen sein. Grundsätzlich kann zwar die Umsetzung auch in aprotischen Lösungsmitteln, wie Dimethylformamid oder Sulfolan, durchgeführt werden, die Aufarbeitung ist aber aufwendiger.

Das im Laufe der Umsetzung und/oder während bzw. nach der Wasserzugabe destillativ zurückgewonnene Lösungsmittel wird den folgenden Ansätzen zugeführt. Gegebenenfalls kann auch aus der salzhaltigen wäßrigen Phase das Lösungsmittel zurückgewonnen werden, dies ist aber meist aufwendiger.

Das erfindungsgemäß hergestellte 1,6-Di($N^3$-cyano-$N^1$-guanidino)hexan wird in üblicher Weise getrocknet, bevorzugt bei 70 - 90 °C im Vakuum, z.B. im Wirbelbett.

Die nachfolgenden Beispiele verdeutlichen die Erfindung:

Beispiel 1 (Vergleichsbeispiel)

Gemäß Beispiel 1 der US-PS 4,537,746 werden 94,5 g (0,5 Mol) 1,6-Hexamethylendiamin-dihydrochlorid und 103 g Natriumdicyanamid mit einem Gehalt von 98 % (1,13 Mol) in 700 ml n-Butanol suspendiert. Die Mischung wird 8,5 Stunden am Rückfluß gekocht. Nach dem Abkühlen wird der Feststoff abfiltriert und

mit Eiswasser gewaschen. Das Produkt wird aus wäßrigem Methanol (1,5 l Methanol und 2,5 l Wasser) umkristallisiert und getrocknet. Es werden 55,8 g, entsprechend 44,6 % der Theorie (bezogen auf Hexamethylendiamin-dihydrochlorid), 1,6-Di ($N^3$-cyano-$N^1$-guanidino)hexan erhalten mit einer Reinheit von 93,5 % (HPLC-Analyse).

Der Ansatz wurde wiederholt, wobei jedoch bei sonst gleichen Bedingungen 90,8 g (1,0 Mol) Natriumdicyanamid (98%ig) eingesetzt wurden. Statt der Umkristallisierung wurde das Rohprodukt in 500 ml Ethanol/Wasser = 1:1 bei 45 °C ausgerührt und abfiltriert. Die Ausbeute betrug 79,6 g (63,7 % der Theorie), die Reinheit 94 %.

Beispiel 2 (Vergleichsbeispiel)

Angelehnt an das Verfahren gemäß Beispiel 1 werden Diaminohexan-dihydrochlorid und Natriumdicyanamid in genau stöchiometrischem Verhältnis eingesetzt und umgesetzt:
1,6-Diaminohexan-dihydrochlorid 23,6 kg (125 Mol)
Natriumdicyanamid (98%ig) 22.7 kg (250 Mol)
n-Butanol 175 l
Reaktionszeit 8 Stunden
Reaktionstemperatur 115-120 °C

Nach beendeter Umsetzung wird abgekühlt und zentrifugiert, anschließend mit kaltem Wasser (ca. 100 l) gewaschen und erneut zentrifugiert. Da das Produkt sehr feinkörnig anfällt, erfordert die Zentrifugation mehrere Stunden.

Nach dem Trocknen erhält man 24,9 kg 1,6-Di($N^3$-cyano-$N^1$-guanidino)hexan, entsprechend einer Ausbeute von 79,7 % der Theorie, mit einem Schmelzpunkt von 200-202 °C und einer Reinheit von 92 % (HPLC-Analyse); Korngröße: 50-100 µm (Hauptanteil) / Rasterelektronenmikroskop.

Beispiel 3

71,7 g (0,556 Mol) 1,6-Diaminohexan (90 %ige wäßrige Lösung) werden zu 900 ml n-Butanol gegeben; unter Rühren fügt man soviel konzentrierte Salzsäure zu (ca. 90 ml), bis der pH etwa 6 beträgt. Anschließend trägt man 105 g (2 x 0,556 Mol) Natriumdicyanamid (94 %ig, Restfeuchte 6 %) ein und gibt unter pH-Messung (Glaseleketrode) bei etwa 25 °C 5 ml Triethylamin zu, wobei sich ein pH-Wert von 9,2 einstellt. Dieses Reaktionsgemisch wird erwärmt, und ab ca. 97 °C werden ca. 600 ml eines Butanol-Wasser-Gemisches abdestilliert. Nach weiterem Erwärmen wird 3 Stunden am Rückfluß (113 - 115 °C) erhitzt - das Reaktionsgemisch enthält hierbei 2 - 4 Gew.-% Wasser, bezogen auf das anwesende Lösungsmittel einschließlich Wasser. Anschließend wird ein Teil Butanol abdestilliert und bereits in der Siedehitze langsam 350 ml Wasser zugegeben, wobei das Gemisch abkühlt, Butanol-Wasser-Azeotropgemisch abdestilliert und sich das gewünschte Reaktionsprodukt in Form grober, leicht filtrierbarer Teilchen ausbildet. Der Niederschlag wird nach Abkühlen auf 40 - 50 °C abgesaugt und im Vakuum bei 90 °C getrocknet. Erhalten werden 120 g (=86,3 % der Theorie) 1,6-Di($N^3$-cyano-$N^1$-guanidino)hexan mit einem Schmelzpunkt von 208-210 °C und einer durch HPLC-Analyse bestimmten Reinheit von 96,3 %.

Beispiel 4

Beispiel 3 wird wiederholt, wobei aber als katalytisch wirksame Base Pyridin zugegeben (ca. 10 ml) und ein pH-Wert von 9 eingestellt wird.
Erhalten werden 119 g (=85,6 % der Theorie) 1,6-Di($N^3$-cyano-$N^1$-guanidino)hexan mit einem Schmelzpunkt von 207 - 210 °C und einer Reinheit (HPLC) von 95,4 %.

Beispiel 5

In einem 2 l-Dreihalskolben mit Rührer, Thermometer und Destillationsaufsatz werden 105 g (1,112 Mol) Natriumdicyanamid (94 %ig / 6 x Feuchte) in 900 ml n-Butanol vorgelegt, mit 5 ml Triethylamin versetzt und dann auf 80 °C erwärmt. Hierzu wird eine wäßrige, pH 5,5 aufweisende Lösung, erhalten aus 64,5 g (0,556 Mol) 1,6-Diaminohexan (100 %ig) und 90 ml Salzsäure, zugegeben. Beim weiteren Erwärmen werden 600 ml Butanol-Wasser-Gemisch abdestilliert und 3 Stunden am Rückfluß,zuletzt bei 117 -118 °C, gekocht. Die Zugabe von Wasser (350 ml) in der Siedehitze und weitere Aufarbeitung werden gemäß Beispiel 3 ausgeführt. Erhalten werden 119 g (=85,6 % der Theorie) 1,6-Di($N^3$-cyano-$N^1$-guanidino)hexan mit einem Schmelzpunkt von 208 - 209 °C, HPLC-Reinheit 95,9 %.

Beispiel 6

In einem 1,2 $m^3$-Rührkessel werden 320 kg einer 90 gew.-%igen wäßrigen Lösung von 1,6-Diaminohexan (2,48 kMol) mit ca. 400 l Salzsäure (2 x 2,48 kMol) auf pH 5,5 eingestellt. Unter Normaldruck werden 190 l Wasser abdestilliert.
In einem 6 $m^3$-Rührkessel werden 4500 l n-Butanol, 25 kg Triethylamin und 465 kg Natriumdicyanamid (95 %ig, 2 x 2,48 kMol) vorgelegt. Hierzu wird die oben hergestellte Lösung von Hexamethylendiamin-dihydrochlorid zugepumpt. Beim Erwärmen auf Reaktionstemperatur werden unter vermindertem Druck ca. 2000 l Butanol-Wasser-Gemisch abgezogen. Während der weiteren Umsetzung unter Rückfluß (113 - 117 °C) werden über einen Zeitraum von zwei Stunden nochmals 1000 l n-Butanol unter Normaldruck abdestilliert. Bereits in der Siedehitze werden langsam 3000 l Wasser zugegeben, wobei noch vorhandenes Butanol als Azeotrop mit Wasser abdestilliert. Nach Abkühlen auf 40 - 50 °C wird der vorhandene Feststoff abzentrifugiert.

Das Produkt läßt sich problemlos in kurzer Zeit filtrieren.

Erhalten werden 550 kg (= 88,7 % der Theorie) 1,6-Di($N^3$-cyano-$N^1$-guanidino)hexan; Schmelzpunkt 207 - 209 °C;
Reinheit (HPLC) 96,4 % .
Korngröße: 100-250 µm(Agglomerate) / Rasterelektronenmikroskop.

Beispiel 7

Beispiel 6 wird wiederholt, wobei gleiche Mengen eingesetzt und während des Erwärmens unter vermindertem Druck 2000 l Butanol-Wasser-Gemisch abdestilliert und anschließend innerhalb von etwa 2,5 Stunden weitere 2000 l Butanol unter Normaldruck abdestilliert werden.
Das verbleibende Reaktionsgemisch wird mit 3000 l Wasser versetzt und restliches Butanol bis zu einer Kopftemperatur von ca. 100 °C abgezogen. Nach Abkühlen auf 40 - 50 °C wird zentrifugiert und der Feststoff bei 90 °C im Vakuum getrocknet.
Erhalten werden 590 kg, entsprechend 95 % der Theorie, 1,6-Di($N^3$-cyano-$N^1$-guanidino)hexan; Schmelzpunkt 208 -210 °C, Reinheit (HPLC-Analyse) 95,8 %.
Korngröße: 100-250 µm (Agglomerate) / Rasterelektronenmikroskop.

Beispiel 8

Beispiel 7 wird wiederholt, wobei jedoch aus Beispiel 7 redestilliertes Butanol eingesetzt wird. Triethylamin braucht nicht ergänzt zu werden, weil dieses mit dem n-Butanol abdestilliert und damit in diesem enthalten ist. Das Produkt wird in 93,5 %iger Ausbeute erhalten; Gehalt 95,3%; Schmelzpunkt 207 - 210 °C.

## Patentansprüche

**1.** Verfahren zur Herstellung von 1,6-Di($N^3$-cyano-$N^1$-guanidino)hexan durch Umsetzung eines Alkalidicyanamids mit einem Hexamethylendiammoniumsalz in alkoholischer Lösung bzw. Suspension unter Erwärmen auf mindestens 80 °C, Abkühlen des Reaktionsgemisches nach beendeter Umsetzung, Abtrennen des auskristallisierten und durch Behandlung mit Wasser im wesentlichen salzfrei gemachten Reaktionsproduktes und Trocknen desselben,
dadurch gekennzeichnet,
daß man die Umsetzung in Gegenwart von 0,1 bis 20 Gew.-% Wasser, bezogen auf das im Reaktionsgemisch anwesende Lösungsmittel einschließlich Wasser, und einer katalytischen Menge einer Base aus der Reihe aliphatisches oder cycloaliphatisches tertiäres Amin, N-heterocyclische Base oder Hexamethylendiamin durchführt, wobei eine solche Menge Base eingesetzt wird, daß das die Reaktionspartner in stöchiometrischem Verhältnis enthaltende Reaktionsgemisch zu Beginn der Umsetzung einen pH-Wert von 8 bis 10 - gemessen mit einer Glaselektrode bei etwa 25 °C - aufweist, nach beendeter Umsetzung das Reaktionsgemisch oberhalb 80 °C mit Wasser versetzt und das auskristallisierte Reaktionsprodukt von der salzhaltigen flüssigen Phase abtrennt.

**2.** Verfahren nach Anspruch 1, dadurch gekennzeichnet, daß man Natriumdicyanamid verwendet.

**3.** Verfahren nach Anspruch 1 oder 2, dadurch gekennzeichnet, daß man Hexamethylendiamin-dihydrochlorid als Hexamethylendiammoniumsalz verwendet

4. Verfahren nach Anspruch 3, dadurch gekennzeichnet, daß man eine wäßrige oder wäßrig-alkoholische Lösung von in situ aus Hexamethylendiamin und wäßriger Salzsäure, gegebenenfalls in Gegenwart eines Alkohols, gebildetem Hexamethylendiamin-dihydrochlorid verwendet.

5. Verfahren nach einem der Ansprüche 1 bis 4, dadurch gekennzeichnet, daß man einen oder mehrere mono- oder divalente Alkohole mit 1 bis 6 C-Atomen, bevorzugt einen primären monovalenten Alkohol mit 3 bis 5 C-Atomen, zur Herstellung des Reaktionsgemisches einsetzt.

6. Verfahren nach Anspruch 5, dadurch gekennzeichnet, daß man n-Butanol verwendet.

7. Verfahren nach einem der Ansprüche 1 bis 6,
dadurch gekennzeichnet,
daß man die Umsetzung bei Reaktionstemperaturen oberhalb etwa 100 °C in Gegenwart von 0,5 bis 5 Gew.-% Wasser, bezogen auf das im Reaktionsgemisch anwesende Lösungsmittel einschließlich Wasser durchführt.

8. Verfahren nach einem der Ansprüche 1 bis 7, dadurch gekennzeichnet, daß man ein Reaktionsgemisch mit einem Wassergehalt von 5 bis 20 Gew.-%, bezogen auf anwesendes Lösungsmittel einschließlich Wasser, herstellt und zu Beginn der Umsetzung den Wassergehalt durch Abdestillieren von Wasser, gegebenenfalls als Azeotrop mit dem Alkohol, auf 0,1 bis 5 Gew.-%, bezogen auf anwesendes Lösungsmittel einschließlich Wasser, erniedrigt.

9. Verfahren nach einem der Ansprüche 1 bis 8,
dadurch gekennzeichnet,
daß man als Base ein aliphatisches oder cycloaliphatisches tertiäres Amin, oder eine N-heterocyclische Base mit jeweils 5 bis 7 C-Atomen und 1 oder 2 N-Atomen verwendet.

10. Verfahren nach einem der Ansprüche 1 bis 9, dadurch gekennzeichnet, daß man den pH-Wert des Reaktionsgemisches zu Beginn der Umsetzung auf einen Wert um 9 bis 9,5 einstellt - gemessen mit einer Glaselektrode bei etwa 25 °C.

11. Verfahren nach einem der Ansprüche 1 bis 10, dadurch gekennzeichnet, daß man die Umsetzung durch Erwärmen auf 80 °C bis 170 °C, bevorzugt 110 °C bis 150 °C, durchführt.

12. Verfahren nach einem der Ansprüche 1 bis 11, dadurch gekennzeichnet, daß man nach beendeter Umsetzung, vorzugsweise nach Entfernen eines Teils des alkoholischen Lösungsmittels durch Abdestillieren, das Reaktionsgemisch oberhalb 80 °C mit Wasser versetzt, noch vorhandenes Lösungsmittel allein oder als Azeotrop mit Wasser gegebenenfalls abdestilliert, auf etwa 25 °C bis 60 °C, bevorzugt 30 °C bis 50 °C, abkühlt und bei dieser Temperatur die kristalline von der flüssigen Phase trennt.

13. Verfahren nach den Ansprüchen 2, 4, 6, 8, 10 und 12, dadurch gekennzeichnet, daß man die Umsetzung bei im wesentlichen 110 bis 120 °C durchführt, Triethylamin als Katalysator verwendet, nach beendeter Umsetzung einen Teil des n-Butanols abdestilliert und nach Zugabe von Wasser noch vorhandenes n-Butanol als Azeotrop mit Wasser abdestilliert.

**Claims**

1. Process for the preparation of 1,6-di($N^3$-cyano-$N^1$-guanidino)hexane by reacting an alkali metal dicyanamide with a hexamethylenediammonium salt in alcoholic solution or suspension with warming to at least 80 °C, cooling the reaction mixture after completion of the reaction, separating off the crystallized reaction product which has been rendered essentially salt-free by treatment with water and drying same, characterized in that the reaction is carried out in the presence of 0.1 to 20% by weight of water, based on the solvent present in the reaction mixture inoluding water, and a catalytic amount of a base selected from the group consisting of aliphatic or cycloaliphatic tertiary amine, N-heterocyclic base or hexamethylenediamine, such an amount of base being employed that the reaction mixture containing the reaction components in stoichiometric proportions has a pH - measured using a glass

electrode at about 25 ° C - of 8 to 10 at the beginning of the reaction, water being added to the reaction mixture above 80 ° C after completion of the reaction and the crystallized reaction product being separated from the saltcontaining liquid phase.

2. Process according to Claim 1, characterized in that sodium dicyanamide is used.

3. Process according to Claim 1 or 2, characterized in that hexamethylenediamine dihydrochloride is used as the hexamethylenediammonium salt.

4. Process according to Claim 3, characterized in that an aqueous or aqueous-alcoholic solution of hexamethylenediamine dihydrochloride formed in situ from hexamethylenediamine and aqueous hydrochloric acid, optionally in the presence of an alcohol, is used.

5. Process according to one of Claims 1 to 4, characterized in that one or more mono- or divalent alcohols have 1 to 6 C-atoms, preferably a primary monovalent alcohol having 3 to 5 C-atoms, is/are employed for the preparation of the reaction mixture.

6. Process according to Claim 5, characterized in that N-butanol is used.

7. Process according to one of Claims 1 to 6, characterized in that the reaction is carried out in the presence of 0.5 to 5% by weight of water, based on the solvent present in the reaction mixture including water, at reaction temperatures above about 100 ° C.

8. Process according to one of Claims 1 to 7, characterized in that a reaction mixture having a water content from 5 to 20% by weight, based on solvent present including water, is prepared and at the beginning of the reaction, the water content is reduced by distilling off water, optionally as an azeotrope with the alcohol, to 0.1 to 5% by weight, based on solvent present including water.

9. Process according to one of Claims 1 to 8, characterized in that an aliphatic or cycloaliphatic tertiary amine or an N-heterocyclic base each having 5 to 7 carbon atoms and 1 or 2 nitrogen atoms is used as the base.

10. Process according to one of Claims 1 to 9, characterized in that the pH of the reaction mixture at the beginning of the reaction is adjusted to a value around 9 to 9.5 - measured using a glass electrode at about 25 ° C.

11. Process according to one of Claims 1 to 10, characterized in that the reaction is carried out by warming to 80 ° C to 170 ° C, preferably 110 ° C to 150 ° C.

12. Process according to one of Claims 1 to 11, characterized in that after completion of the reaction, preferably after removing a part of the alcoholic solvent by distilling off, water is added to the reaction mixture above 80 ° C, solvent still present is distilled off alone or as an azeotrope with water, the mixture is cooled to about 25 ° C to 60 ° C, preferably 30 ° C to 50 ° C and the crystalline phase is separated from the liquid phase at this temperature.

13. Process according to Claims 2, 4, 6, 8, 10 and 12, characterized in that the reaction is carried out substantially at 110 to 120 ° C, triethylamine is used as the catalyst, a part of the n-butanol is distilled off after completion of the reaction and, after addition of water, n-butanol still present is distilled off as an azeotrope with water.

**Revendications**

1. Procédé pour la préparation du 1,6-di($N^3$-cyano-$N^1$-guanidino)-hexane par réaction d'un dicyanamidure alcalin avec un sel d'hexaméthylènediammonium en solution ou suspension alcoolique, par chauffage à au moins 80 ° C, refroidissement du mélange réactionnel une fois la réaction terminée, séparation du produit de réaction cristallisé et rendu pratiquement exempt de sel par traitement par l'eau, et séchage de celui-ci, caractérisé en ce que l'on effectue la réaction en présence de 0,1 à 20 % en poids d'eau, par rapport au solvant, eau comprise, présent dans le mélange réactionnel, et d'une quantité catalytique

d'une base choisie parmi une amine tertiaire aliphatique ou cycloaliphatique, une base N-hétérocyclique ou l'hexaméthylènediamine, en utilisant une quantité de base telle que le mélange réactionnel, contenant les partenaires réactionnels en proportions stoechiométriques, présente au début de la réaction un pH de 8 à 10 - mesuré aux environs de 25° C à l'aide d'une électrode en verre - une fois la réaction terminée, on ajoute de l'eau au mélange réactionnel au-dessus de 80° C, et on sépare de la phase liquide contenant des sels le produit réactionnel cristallisé.

**2.** Procédé selon la revendication 1, caractérisé en ce que l'on utilise le dicyanamidure de sodium.

**3.** Procédé selon la revendication 1 ou 2, caractérisé en ce que l'on utilise en tant que sel d'hexaméthylènediammonium, le dichlorhydrate d'hexaméthylènediamine.

**4.** Procédé selon la revendication 3, caractérisé en ce que l'on utilise une solution aqueuse ou aqueuse-alcoolique de dichlorhydrate d'hexaméthylènediamine formé in situ à partir d'hexaméthylènediamine et d'acide chlorhydrique en solution aqueuse, éventuellement en présence d'un alcool,

**5.** Procédé selon l'une quelconque des revendications 1 à 4, caractérisé en ce que l'on utilise pour la préparation du mélange réactionnel un ou plusieurs alcools mono- ou divalents ayant de 1 à 6 atomes de carbone, de préférence un alcool monovalent primaire ayant de 3 à 5 atomes de carbone.

**6.** Procédé selon la revendication 5, caractérisé en ce eue l'on utilise le n-butanol.

**7.** Procédé selon l'une quelconque des revendications 1 à 6, caractérisé en ce que l'on effectue la réaction à des températures de réaction supérieures à environ 100° C, en présence de 0,5 à 5 % en poids d'eau, par rapport au solvant présent dans le mélange réactionnel, eau comprise.

**8.** Procédé selon l'une quelconque des revendications 1 à 7, caractérisé en ce que l'on prépare un mélange réactionnel ayant une teneur en eau de 5 à 20 % en poids, par rapport au solvant présent, eau comprise, et au début de la réaction, on abaisse la teneur en eau jusqu'à 0,1 à 5 % en poids, par rapport au solvant présent, eau comprise, par élimination de l'eau par distillation, éventuellement sous forme d'azéotrope, avec l'alcool.

**9.** Procédé selon l'une quelconque des revendications 1 à 8, caractérisé en ce que l'on utilise en tant que base une amine tertiaire aliphatique ou cyclo-aliphatique ou une base N-hétérocyclique ayant chacune de 5 à 7 atomes de carbone et 1 ou 2 atomes d'azote.

**10.** Procédé selon l'une quelconque des revendications 1 à 9, caractérisé en ce que l'on ajuste le pH du mélange réactionnel, au début de la réaction, à une valeur d'environ 9 à 9,5 - mesurée avec une électrode en verre aux environs de 25° C,

**11.** Procédé selon l'une quelconque des revendications 1 à 10, caractérisé en ce que l'on effectue la réaction par chauffage à 80-170° C, de préférence à 110-150° C.

**12.** Procédé selon l'une quelconque des revendications 1 à 11, caractérisé en ce que, une fois la réaction terminée, de préférence après élimination d'une partie du solvant alcoolique par distillation, on ajoute de l'eau au mélange réactionnel au-dessus de 80° C, éventuellement on élimine par distillation le solvant encore présent, seul ou sous forme d'azéotrope avec l'eau, on refroidit aux environs de 25 à 60° C, de préférence de 30 à 50° C, et on sépare à cette température la phase cristalline de la phase liquide.

**13.** Procédé selon l'une quelconque des revendications 2, 4, 8, 10 et 12, caractérisé en ce que l'on effectue la réaction essentiellement à 110-120° C, on utilise la triéthylamine en tant que catalyseur, une fois la réaction terminée, on élimine par distillation une partie du n-butanol, et après addition d'eau on élimine par distillation le n-butanol encore présent, sous forme d'azéotrope avec l'eau.